# EUROPÄISCHE PATENTSCHRIFT

(11) **EP 1 646 822 B1**
(45) Veröffentlichungstag und Bekanntmachung des Hinweises auf die Patenterteilung: **21.10.2009**
(21) Anmeldenummer: 04741298.6
(22) Anmeldetag: 22.07.2004
(51) Int. Cl.: F16M 11/40, A61B 19/00

(54) **GELENKKETTENVORRICHTUNG**
LINK CHAIN DEVICE
DISPOSITIF DE CHAINE A ARTICULATIONS

(30) Priorität: 23.07.2003 DE 10334135
(43) Veröffentlichungstag der Anmeldung: 19.04.2006
(73) Patentinhaber: Forschungsverbund Berlin e.V., 12489 Berlin (DE)
(72) Erfinder: MEYER, Bernhard, 81549 München (DE); MÜLLER, Ingo, 13403 Berlin (DE); MUSOLFF, André, 12205 Berlin (DE); SCHALLER, Karl, 53227 Bonn (DE); SAHOTA, Harsimar, 80992 Berlin (DE); SPREKELS, Jürgen, 14199 Berlin (DE)
(74) Vertreter: Theobald, Andreas
(86) Internationale Anmeldenummer: PCT/EP2004/008432
(87) Internationale Veröffentlichungsnummer: WO 2005/010425

(56) Entgegenhaltungen:
- DE-C- 19 916 244
- US-A- 3 529 797
- US-A- 5 624 381
- US-A- 5 873 817
- US-A- 6 013 027
- US-A1- 2002 058 860

## Beschreibung

Die vorliegende Erfindung betrifft eine Gelenkkettenvorrichtung, die in einen fixierten Zustand und in einen flexiblen Zustand gebracht werden kann.

Gattungsgemäße Gelenkkettenvorrichtungen finden u.a. in Haltesystemen zum Halten von Gegenständen wie Lampen, Mikrofonen, etc. Anwendung. In medizinischen Haltesystemen sollen bspw. über eine an der Spitze der Gelenkkette angebrachte Universalklammer (neuro-) chirurgische Instrumente im Bereich eines operativen Zugangs positioniert werden. Um ein genaues Positionieren zu ermöglichen, muss die Gelenkkette zunächst so lange flexibel sein, bis der Arzt oder eine Hilfsperson das Instrument in die gewünschte Position gebracht hat. Anschließend soll die Gelenkkette steif sein, damit die eingestellte Position während des operativen Eingriffs beibehalten wird.

Ein Haupteinsatzbereich der beschriebenen medizinischen Haltesysteme liegt in der Neurochirurgie bei kranialen Operationen, d.h. bei Operationen im Bereich des Groß- oder des Kleinhirns, bei denen Teile der Hirnlappen bzw. die beiden Großhirn- oder Kleinhirnhemisphären zur Seite oder auseinander gehalten werden müssen, um die anatomische Zielregion der Operation zugänglich zu machen. An der Universalklammer werden zu diesem Zweck spezielle flache sog. Hirnspatel, die meist aus Metall bestehen und die in ihrer Form dem bekannten Mundspatel aus Holz ähneln, befestigt.

Ein medizinisches Haltesystem mit einer Gelenkkette ist bspw. unter der Bezeichnung "DORO Reaktor System" der Firma "pro medical instruments GmbH" bekannt. Durch die Gelenkkette dieses Haltesystems erstreckt sich ein Spanndraht, der mittels einer Handkurbel gespannt werden kann. Im gespannten Zustand werden die Gelenkglieder der Gelenkkette aneinander gedrückt, so dass sich die Gelenkkette aufgrund der Reibung zwischen den Gelenkgliedern versteift. Die Betätigung der Handkurbel erfolgt dabei durch den Assistenten des Chirurgen. Aufgrund des mechanischen Bewegens der Handkurbel kann es zu Erschütterungen am Operationstisch und damit an der Operationsstelle, dem sog. Operationssitus, selbst kommen. Daher muss der Chirurg während des Betätigens der Handkurbel den Spatel mit seiner Arbeitshand sichern und sich ständig mit seinem Assistenten abstimmen. Neben den Positionswechseln des Spatels unterbrechen so auch das Verstärken oder Lockern der Verspannung der Gelenkkette den natürlichen Ablauf der Operation.

Die US 2002/058860 A1 beschreibt eine Gelenkkettenvorrichtung in Form eines medizinischen Instrumenthalters, welche mit einer Anzahl Gelenkglieder, einem sich durch die Gelenkglieder erstreckenden Spanndraht und einer Spannvorrichtung ausgestattet ist. Die Spannvorrichtung befindet sich am proximalen Ende des Spanndrahtes und weist am Spanndraht angreifende Formgedächtnisdrähte auf, die bei Stromfluss kontrahieren und so auf den Spanndraht zum Spannen der Gelenkkette einwirken.

Die DE 199 16 244 C1 beschreibt einen Bowdenzug, dessen Seele zumindest teilweise und vorzugsweise vollständig aus einer Formgedächtnislegierung besteht. Die Formgedächtnislegierung kommt dabei zur Anwendung, um die beim Einsatz des Bowdenzugs auftretende Zugspannung besser zu begrenzen.

Die US 5,624,381 beschreibt ein medizinisches Instrument mit Gelenkgliedern und einem sich durch die Gelenkglieder erstreckenden Spannseil. Es ist erwähnt, dass die Gelenkglieder auch aus einer Formgedächtnislegierung hergestellt sein können.

Aufgabe der vorliegenden Erfindung ist es, eine verbesserte Gelenkkettenvorrichtung sowie ein verbessertes Haltesystem zu schaffen, mit denen mechanische Erschütterungen beim Herstellen oder Lösen der Spannung reduziert werden können.

Diese Aufgabe wird durch eine Gelenkkettenvorrichtung nach Anspruch 1 sowie durch ein Haltesystem nach Anspruch 7 gelöst. Die abhängigen Ansprüche enthalten vorteilhafte Ausgestaltungen der Erfindung.

Eine erfindungsgemäße Gelenkkettenvorrichtung weist eine Anzahl von Gelenkgliedern, welche eine Gelenkkette bilden, und eine Spanneinrichtung, welche mindestens ein sich durch die Gelenkglieder erstreckendes Spannseil oder mindestens einen sich durch die Gelenkglieder erstreckenden Spanndraht umfasst, auf. Die Gelenkkette kann durch Spannen des Spannseils oder -drahtes in einen fixierten Zustand, in dem benachbarte Gelenkglieder im Wesentlichen relativ zueinander fixiert sind, und durch Entspannen des Spannseils oder -drahtes in einen flexiblen Zustand, in dem sich benachbarte Gelenkglieder relativ zueinander bewegen lassen, gebracht werden. Unter "im Wesentlichen zueinander fixiert" soll hierbei zu verstehen sein, dass die Kraft, die im fixierten Zustand zum Bewegen der Gelenkglieder nötig ist, die Kraft, die im flexiblen Zustand nötig ist, deutlich übersteigt. Die erfindungsgemäße Gelenkkettenvorrichtung ist so gestaltet, dass die Spanneinrichtung ein Formgedächtniselement umfasst, welches derart ausgestaltet und angeordnet ist, dass der fixierte Zustand durch Ändern des Formzustandes des Formgedächtniselementes herbeizuführen oder aufzuheben ist. Als Formgedächtniselement ist hierbei ein Element anzusehen, das aus einem Material mit Formgedächtnis hergestellt ist, d.h. aus einem Material, das bei einer bestimmten Temperatur einen Übergang von einem Formzustand in einen anderen Formzustand ausführt, der über die übliche temperaturbedingte Ausdehnung/Kontraktion des Materials hinausgeht.

In der erfindungsgemäßen Gelenkkettenvorrichtung ist das sich durch die Gelenkglieder erstreckende Spannseil oder der sich durch die Gelenkglieder erstreckende Spanndraht als Formgedächtnisdraht ausgebildet. Der Formgedächtnisdraht bildet in diesem Fall das Formgedächtniselement der Spanneinrichtung. Formgedächtnisdrähte ändern ihre Länge als Reaktion auf eine Änderung der Temperatur. So kann der Formgedächtnisdraht bspw. durch ein elektrisches Heizen zur Kontraktion gebracht werden. Die Verkürzung des Drahtes führt dabei dazu, dass die Gelenkglieder gegeneinander fixiert werden. Bei Abkühlen des Drahtes dehnt sich dieser wieder aus und stellt so die Flexibilität der Gelenkkette wieder her. Insbesondere kann der Formgedächtnisdraht selbst Teil eines Stromkreises zum elektrischen Heizen des Drahtes sein. Eine mechanische Einrichtung zum Spannen des Drahtes, wie etwa eine Kurbel, ist in dieser Ausgestaltung nicht notwendig.

Besonders vorteilhaft ist es, wenn das Formgedächtniselement aus einer Formgedächtnislegierung mit Zweiweg-Memory-Effekt, im Folgenden Zweiweg-Formgedächtnislegierung genannt, hergestellt ist. Zweiweg-Memory-Effekt bedeutet, dass sich die Formgedächtnislegierung mittels Erwärmen und Abkühlen zwischen zwei Formzuständen hin und her schalten lässt, sich also bspw. bei Erwärmung zusammenzieht und bei Abkühlung wieder ausdehnt. Im Gegensatz dazu muss bei einem Einweg-Memory-Effekt die Formänderung in eine Richtung, bspw. in einen ausgedehnten Zustand, durch eine äußere Kraft herbeigeführt werden, während die Rückkehr in die Ausgangsform, bspw. einen kontrahierten Zustand, durch Ändern der Temperatur der Legierung herbeizuführen ist. Eine Formgedächtnislegierung, die einen Einweg-Memory-Effekt zeigt, wird im Folgenden Einweg-Formgedächtnislegierung genannt. Der Zweiweg-Memory-Effekt bietet den Vorteil, dass das Ändern der Form des Formgedächtniselementes in beide Richtungen, und damit sowohl das Versteifen als auch das Lösen der Gelenkglieder voneinander, ohne mechanische Hilfsmittel zu bewerkstelligen ist.

Zum Ändern der Form des Formgedächtniselementes kann beispielsweise mittels elektrischer Beheizung eine Temperaturänderung des Formgedächtniselementes herbeigeführt werden, auf die es mit der Änderung seines Formzustandes reagiert. Die Änderung des Formzustandes des Formgedächtniselementes erfolgt ruckfrei und hilft so, Erschütterungen im Operationsbereich zu vermeiden.

Wenn die Gelenkglieder Metall umfassen, und insbesondere, wenn sie vollständig aus Metall hergestellt sind, bietet sich die Möglichkeit, den Stromkreis zum Heizen des Formgedächtnisdrahts bei entsprechender elektrischer Isolierung des Drahtes gegenüber den Gelenkgliedern über die Gelenkglieder zu schließen.

Die Gelenkkette kann jedoch auch so ausgestaltet sein, dass jedes Gelenkglied mindestens zwei in seiner Axialrichtung verlaufende Durchgangslöcher, bspw. Durchgangsbohrungen, aufweist, und dass das Spannseil oder der Spanndraht mindestens vom proximalen zum distalen Ende der Gelenkkette und wieder zurück geführt ist, so dass der Stromkreis auch geschlossen werden kann, ohne die Gelenkglieder zu verwenden. Dies ermöglicht es, die Gelenkglieder aus einem Isolatormaterial herzustellen.

Als Material für das Formgedächtniselement kann bspw. eine Nickel-Titan(Ni-Ti)-Legierung, eine Kupfer-Aluminium-Nickel(Cu-Al-Ni)-Legierung oder eine Kupfer-Zink-Aluminium(Cu-Zn-Al)-Legierung Verwendung finden oder auch eine beliebige andere Formgedächtnislegierung. Insbesondere die Nickel-Titan-Legierung bietet die Möglichkeit, einen großen Stellweg, d.h. eine große Formänderung, und eine große Kraft beim Ändern der Form zu realisieren. Zudem weist die Nickel-Titan-Legierung eine hohe Langzeitstabilität auf. Außer aus den genannten Materialien kann das Formgedächtniselement jedoch auch oder aus einem anderen Formgedächtnismaterial hergestellt sein. In Frage kommen neben Legierungen bspw. auch Formgedächtniskunststoffe.

Um das Eigengewicht der Gelenkkette niedrig zu halten, können die Gelenkglieder aus Kunststoff hergestellt sein. Ggf. können in den Kunststoff elektrische Leiter integriert sein, die das Herstellen eines das Spannseil oder den Spanndraht und die Gelenkglieder umfassenden Stromkreises ermöglichen.

Um das Fixieren der Gelenkglieder relativ zueinander zu unterstützen, können diese an denjenigen Flächen, die benachbarten Gelenkgliedern zugewandt sind, eine reibungsverstärkende Oberflächenstruktur aufweisen. Die reibungsverstärkende Oberflächenstruktur kann dabei bspw. durch Beschichten oder Aufrauhen der Oberfläche erzeugt werden.

Ein erfindungsgemäßes Haltesystem, insbesondere für medizinische Zwecke, umfasst eine erfindungsgemäße Gelenkkettenvorrichtung, ein Halteelement, das zum Halten eines Gegenstandes, insbesondere eines medizinischen Instrumentes oder Werkzeuges, ausgebildet ist, sowie eine Einrichtung zum Heizen und/oder Kühlen des Formgedächtniselementes. Die Einrichtung zum Heizen und/oder Kühlen des Formgedächtniselementes kann insbesondere eine elektrische Heizung umfassen.

Weitere Merkmale, Eigenschaften und Vorteile der vorliegenden Erfindung ergeben sich aus der nachfolgenden Beschreibung von Ausführungsbeispielen, wobei auf die beiliegenden Zeichnungen Bezug genommen wird.
- Fig. 1: zeigt ein erstes Ausführungsbeispiel für die erfindungsgemäße Gelenkkettenvorrichtung.
- Fig. 2: zeigt ein Gelenkglied des ersten Ausführungsbeispiels in einer schematischen Seitenansicht.
- Fig. 3: zeigt ein Gelenkglied des ersten Ausführungsbeispiels in einer schematischen Vorderansicht.
- Fig. 4: zeigt eine schematische Darstellung der Abhängigkeit der Länge des Formgedächtnisdrahtes des ersten Ausführungsbeispiels von seiner Temperatur.

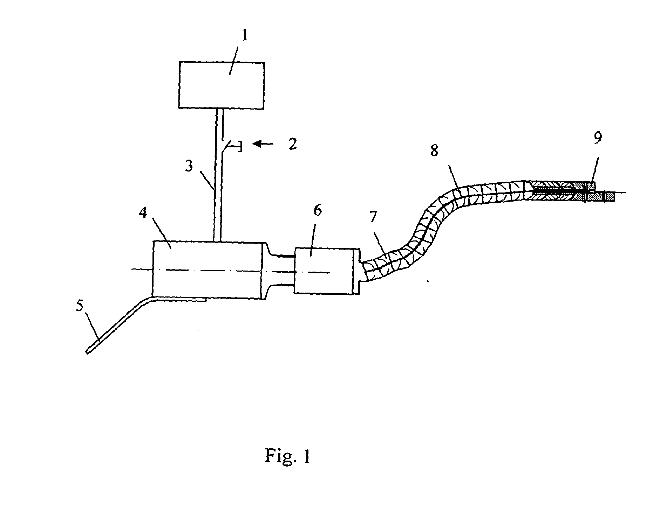

Als ein erstes Ausführungsbeispiel für die Erfindung ist in der Figur 1 eine chirurgische Haltevorrichtung mit einer erfindungsgemäßen Gelenkkettenvorrichtung dargestellt.

Die Haltevorrichtung umfasst eine Gelenkkette 7, die aus einer Anzahl von Gelenkgliedern 10 aufgebaut ist und an deren einem Ende, dem sog. distalen Ende oder Kettenkopf, eine Universalklammer 9 als Halteelement zum Halten eines in der Figur nicht dargestellten chirurgischen Instrumentes oder Werkzeuges, bspw. einem Hirnspatel, angeordnet ist. Am anderen Ende der Gelenkkette 7, dem sog proximalen Ende oder Kettenfuß, ist die Gelenkkette 7 mit einem Stellring 6 verbunden, der wiederum an einer Steuereinheit 4 befestigt ist. Die Steuereinheit 4 wird von einem Befestigungsstück 5 gehalten, mit dessen Hilfe sich die gesamte Haltevorrichtung bspw. an einem Halterahmen oder Ähnlichem befestigen lässt. Die Steuereinheit 4 wird von einer Stromversorgungseinheit 1 mit Strom versorgt. Mittels eines Fußschalters 2 lässt sich die Stromversorgung ein- und ausschalten.

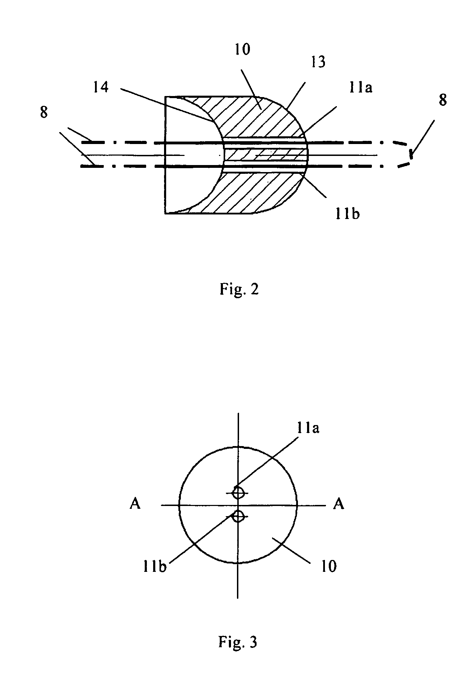

Ein Gelenkglied 10 der Gelenkkette 7 ist in den Figuren 2 und 3 im Detail dargestellt. Die Gelenkglieder 10 können entweder aus Metall oder einem elektrischen Isolator, bspw. Keramik oder Kunststoff, hergestellt sein. Jedes Gelenkglied 10 umfasst eine halbkugelförmige Aussparung 14 sowie einen ebenfalls halbkugelförmigen Vorsprung 13, der am der Aussparung 14 abgewandten Ende des Gelenkgliedes 10 angeordnet ist. Die Abmessungen der Aussparung 14 und des Vorsprungs 13 sind derart gewählt, dass der Vorsprung 13 eines Gelenkgliedes 10 in die Aussparung 14 des benachbarten Gelenkgliedes 10 eingreifen kann.

Jedes Gelenkglied 10 ist in der Nähe seiner Mittelachse A mit zwei in Axialrichtung des Gliedes 10 verlaufenden Durchgangslöchern 11a, 11b versehen, durch die ein Formgedächtnisdraht 8 vom proximalen Ende der Gelenkkette 7 zum distalen Ende der Gelenkkette 7 und wieder zurück freibeweglich geführt ist. Am proximalen Ende ist der Formgedächtnisdraht 8 mit der Steuereinheit 4 elektrisch verbunden.

Als Drahtmaterial kommt im Ausführungsbeispiel eine Nickel-Titan-Legierung als Zweiweg-Formgedächtnis-Legierung zur Anwendung. Es eignen sich jedoch auch andere Zweiweg-Formgedächtnis-Legierungen zum Herstellen des Formgedächtnisdrahtes 8. Die Eigenschaften der verwendeten Nickel-Titan-Legierung sind so gewählt, dass der Formgedächtnisdraht 8 bei einer Temperatur von nicht mehr als ca. 50°C, die insbesondere im Bereich von 40°C bis 50°C und vorzugsweise in der unteren Hälfte dieses Bereiches liegt, zusammengezogen und bei Raumtemperatur ausgedehnt ist.

Die Länge des Formgedächtnisdrahtes 8 bei Raumtemperatur ist derart gewählt, dass die Gelenkglieder 10 frei gegeneinander beweglich sind. Wenn der Formgedächtnisdraht 8 hingegen zusammengezogen ist, drückt er die halbkugelförmigen Vorsprünge 13 der Gelenkglieder 10 in die halbkugelförmigen Aussparung 14. Aufgrund der durch den Druck entstehenden Reibung versteift sich die Gelenkkette 7 und hält das an ihrem distalen Ende befestigte chirurgische Instrument in der Position, in die es vor dem Heizen des Formgedächtnisdrahtes 8 gebracht worden ist. Um die Reibung bei zusammengezogenem Formgedächtnisdraht 8 zu verstärken, kann die Oberfläche der Aussparung 14 oder die des Vorsprunges 13 mit einer reibungsverstärkenden Oberflächenstruktur oder -beschichtung ausgestattet sein. Selbstverständlich können auch beide Oberflächen eine reibungsverstärkende Struktur oder Beschichtung aufweisen.

Das Heizen des Formgedächtnisdrahtes 8 erfolgt elektrisch und wird von der Steuereinheit 4 gesteuert. Wenn sich die Gelenkkette 7 versteifen soll, kann der Arzt die Versorgung der Steuereinheit 4 mit Strom über den Fußschalter 2 einschalten, so dass ein Heizen des Formgedächtnisdrahtes 8 erfolgt. Wenn die Gelenkkette 7 wieder beweglich sein soll, so kann der Arzt die Stromversorgung der Steuereinheit 4 mit Hilfe des Fußschalters 2 wieder unterbrechen.

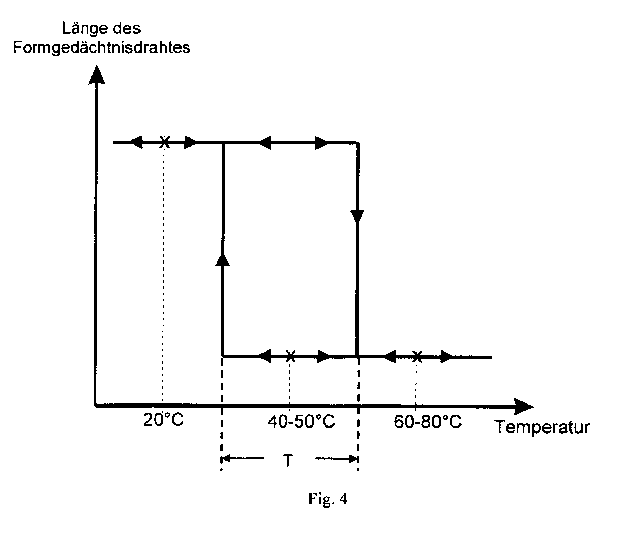

Die Steuerung des elektrischen Heizprozesses geschieht folgendermaßen: Sobald die Stromversorgung aktiviert ist, wird dem Formgedächtnisdraht 8 eine vorbestimmte maximale Leistung zugeführt. Der Formgedächtnisdraht 8 erwärmt sich dabei zunächst auf mehr als ca. 40°C - 50°C, nämlich auf ca. 60°C - 80°C und insbesondere auf ca. 60 - 65°C. Nachdem die Spannung im Formgedächtnisdraht 8 hergestellt ist, fährt die Steuereinheit 4 die Leistung auf einen Wert zurück, der ausreicht, den Formgedächtnisdraht 8 auf einem Wert im Bereich von etwa 40°C bis 50°C, insbesondere auf einen Wert in der unteren Hälfte dieses Bereiches, zu halten. Diese Möglichkeit zur Rückführung bzw. Reduktion der elektrischen Leistung beruht auf einem Verhalten des Formgedächtnisdrahtes 8 bzw. der Formgedächtnislegierung, das als Hysterese bekannt ist und das vereinfacht ausgedrückt besagt, dass in einem bestimmten Temperaturbereich T die Länge des Formgedächtnisdrahtes bei einer bestimmten Temperatur davon abhängt, ob er auf diese Temperatur erwärmt oder abgekühlt wurde. Das Verhalten ist schematisch in Fig. 4 dargestellt. Um den Formgedächtnisdraht 8 maximal zu kontrahieren, findet eine Temperatur oberhalb des Bereiches T Anwendung, die im Ausführungsbeispiel bei ca. 60°C - 80°C liegt. Um den maximal kontrahierten Zustand des Formgedächtnisdrahtes 8 aufrechtzuerhalten genügt jedoch eine niedrigere Temperatur innerhalb des Bereiches T, die im Ausführungsbeispiel bei ca. 40°C - 50°C liegt. Um ein Ausdehnen des Formgedächtnisdrahtes aus dem maximal kontrahierten Zustand zu erreichen, muss die Temperatur auf eine Temperatur unterhalb des Bereiches T gesenkt werden, im Ausführungsbeispiel bspw. auf ca. 20°C.

Der am proximalen Ende der Gelenkkette 7 angeordnete Stellring 6 dient dazu, den Formgedächtnisdraht 8 bei Bedarf vollständig zu lockern. Das vollständige Lockern ist bspw. wichtig, wenn die Gelenkkette 7 sterilisiert werden soll. Die beim Sterilisieren auftretende Temperatur von ca. 135°C würde den Formgedächtnisdraht 8 sonst spannen, wobei sich die Gelenkglieder 10 aufgrund der hohen Temperatur plastisch verformen könnten. Nach dem Sterilisationsprozess kann der Formgedächtnisdraht 8 mit dem Stellring 6 wieder leicht vorgespannt werden. Darüber hinaus dient der Stellring 6 auch zum Nachjustieren des Formgedächtnisdrahtes 8. Das Nachjustieren kann im Laufe der Zeit zum Kompensierens von Alterserscheinungen des Drahtes und/oder der Gelenkglieder 10 sinnvoll sein.

Im ersten Ausführungsbeispiel ist der Formgedächtnisdraht 8 vom proximalen Ende der Gelenkkette 7 bis zum distalen Ende der Gelenkkette und wieder zurück geführt. Alternativ kann jedoch der Formgedächtnisdraht 8 auch nur vom proximalen zum distalen Ende geführt und dort befestigt sein. Falls das Heizen des Formgedächtnisdrahtes 8 elektrisch erfolgt, kann der durch den Formgedächtnisdraht 8 fließende Strom dann über die Gelenkglieder 10 zurückgeführt werden, sofern diese aus Metall hergestellt sind oder metallische Elemente zum Leiten des Stromes umfassen. Alternativ kann das Heizen (ggf. auch das Abkühlen) mittels eines Heizfluids (bzw. Kühlfluids), bspw. Luft, Wasser oder Öl, erfolgen. Die Gelenkglieder können dann vollständig aus einem Isolatormaterial, bspw. Keramik oder Kunststoff, bestehen.

Zwar ist im ersten Ausführungsbeispiel die Formgedächtnislegierung derart gewählt, dass die Kontraktion des Formgedächtnisdrahtes bei ca. 40°C bis 50°C aufrechterhalten werden kann, jedoch ist diese Temperatur nicht zwingend vorgegeben, sondern nur durch die Anwendung im medizinischen Bereich bedingt. Im außermedizinischen Bereich können durchaus auch Formgedächtnislegierungen zur Anwendung kommen, bei denen das Aufrechterhalten der Kontraktion des Formgedächtnisdrahtes deutlich höhere oder niedrigere Temperaturen erfordert. Mögliche Temperaturen für den Übergang von einem Formzustand in einen anderen liegen im Bereich zwischen -120°C und +120°C und hängen von der Zusammensetzung der Formgedächtnislegierung ab.

In allen dargestellten Ausführungsbeispielen stellt die Form der Gelenkglieder nur eine mögliche geeignete Form dar. Die Erfindung ist jedoch nicht auf die dargestellte Form der Gelenkglieder festgelegt, sondern lässt sich auch mit Gelenkketten verwirklichen, deren Gelenkglieder anders geformt sind.

## Patentansprüche

1. Gelenkkettenvorrichtung mit einer Anzahl von Gelenkgliedern (10), welche eine Gelenkkette (7) bilden, und einer Spanneinrichtung, welche ein sich durch die Gelenkglieder (10) erstreckendes Spannseil oder einen sich durch die Gelenkglieder (10) erstreckenden Spanndraht (8) umfasst, wobei die Gelenkkette (7) durch Spannen des Spannseils oder -drahtes (8) in einen fixierten Zustand, in dem benachbarte Gelenkglieder (10) im Wesentlichen relativ zueinander fixiert sind, und durch Entspannen des Spannseils oder -drahtes (8) in einen flexiblen Zustand, in dem sich benachbarte Gelenkglieder (10) relativ zueinander bewegen lassen, gebracht werden kann, wobei die Spanneinrichtung ein Formgedächtniselement (8) umfasst, welches derart ausgestaltet und angeordnet ist, dass der fixierte Zustand durch Ändern eines Formzustandes des Formgedächtniselementes (8) herbeizuführen oder aufzuheben ist **dadurch gekennzeichnet, dass** das Spannseil oder der Spanndraht als Formgedächtnisdraht (8) ausgebildet ist, welcher das Formgedächtniselement der Spanneinrichtung bildet.

2. Gelenkkettenvorrichtung nach Anspruch 1, **dadurch gekennzeichnet, dass** die Gelenkglieder (10) Metall umfassen oder aus Metall gefertigt sind.

3. Gelenkkettenvorrichtung nach Anspruch 1 oder Anspruch 2, **dadurch gekennzeichnet, dass** jedes Gelenkglied (10) mindestens zwei in seiner Axialrichtung verlaufende Durchgangslöcher (11a, 11 b) aufweist, und dass das Spannseil oder der Spanndraht (8) mindestens vom proximalen zum distalen Ende der Gelenkkette und wieder zurück geführt ist.

4. Gelenkkettenvorrichtung nach einem der vorangehenden Ansprüche, **dadurch gekennzeichnet, dass** die Gelenkglieder (10; 110) aus Kunststoff hergestellt sind.

5. Gelenkkettenvorrichtung nach Anspruch 4, **dadurch gekennzeichnet, dass** elektrische Leiter in den Kunststoff integriert sind.

6. Gelenkkettenvorrichtung nach einem der vorangehenden Ansprüche, **dadurch gekennzeichnet, dass** die Gelenkglieder (10; 110) an den einander zugewandten Flächen (13, 14; 113, 114) eine reibungsverstärkende Oberflächenstruktur aufweisen.

7. Haltesystem, insbesondere für medizinische Zwecke, mit einer Gelenkkettenvorrichtung nach einem der vorangehenden Ansprüche, einem Halteelement (9; 109), das zum Halten eines Gegenstandes, insbesondere eines medizinischen Instrumentes oder Werkzeuges, ausgebildet ist, und einer Einrichtung zum Heizen und/oder Kühlen des Formgedächtniselementes (8; 115).

8. Haltesystem nach Anspruch 9, **dadurch gekennzeichnet, dass** die Einrichtung zum Heizen und/oder Kühlen eine elektrische Heizung umfasst.

## Claims

1. A link chain device having a plurality of link elements (10) that form a link chain (7), and having a tensioning device that includes a tensioning cable extending through the link elements (10) or a tensioning wire (8) extending through the link elements (10), wherein the link chain (7) can be brought by tensioning the tensioning cable or wire (8) into a fixed state in which adjacent link elements (10) are substantially fixed in relation to each other, and by relaxing the tensioning cable or wire (8) can be brought into a flexible state in which adjacent link elements (10) can move relative to each other,
wherein the tensioning device includes a shape memory element (8), which is configured and disposed in such a manner that the fixed state can be brought about or cancelled by altering the state of the shape of the shape memory element (8),
**characterised in that** the tensioning cable or tensioning wire is designed as a shape memory wire (8), which forms the shape memory element of the tensioning device.

2. A link chain device according to Claim 1,
**characterised in that** the link elements (10) contain metal or are produced from metal.

3. A link chain device according to Claim 1 or Claim 2,
**characterised in that** each link element (10) comprises at least two through holes (11 a, 11 b) extending in its axial direction,
and **in that** the tensioning cable or the tensioning wire (8) is guided at least from the proximal to the distal end of the link chain and back again.

4. A link chain device according to one of the preceding Claims,
**characterised in that** the link elements (10; 110) are produced from plastic.

5. A link chain device according to Claim 4,
**characterised in that** electric conductors are integrated into the plastic.

6. A link chain device according to one of the preceding Claims,
**characterised in that** the link elements (10; 110) comprise a friction-reinforcing surface structure on the surfaces facing each other (13; 14; 113, 114).

7. A holding system, especially for medical purposes, having a link chain device according to one of the preceding Claims, a holding element (9; 109) designed to hold an object, especially a medical instrument or tool, and a device for heating and/or cooling the shape memory element (8; 115).

8. A holding system according to Claim 9,
**characterised in that** the device for heating and/or cooling includes a filament heating.

## Revendications

1. Dispositif à chaîne articulée comportant un certain nombre d'éléments d'articulation (10) formant une chaîne articulée (7), et un dispositif de réglage de la tension qui comprend un câble tendeur passant à travers les éléments d'articulation (10) ou un fil tendeur (8) passant à travers les éléments d'articulation (10), la chaîne articulée (7) pouvant être amenée par serrage du câble tendeur ou du fil tendeur (8) dans une position fixe dans laquelle les éléments d'articulation (10) adjacents sont sensiblement bloqués les uns par rapport aux autres, et par desserrage du câble tendeur ou du fil tendeur (8) dans une position flexible dans laquelle les éléments d'articulation (10) adjacents sont mobiles les uns par rapport aux autres, le dispositif de réglage de la tension comprenant un élément à mémoire de forme (8), qui est conçu et disposé de telle sorte que la position fixe peut être provoquée ou supprimée en modifiant l'état de la forme de l'élément à mémoire de forme (8), **caractérisé en ce que** le câble tendeur ou le fil tendeur est conçu sous forme d'un fil à mémoire de forme (8) qui constitue l'élément à mémoire de forme (8) du dispositif de réglage de la tension.

2. Dispositif à chaîne articulée selon la revendication 1, **caractérisé en ce que** les éléments d'articulation (10) comportent du métal ou sont fabriqués en métal.

3. Dispositif à chaîne articulée selon la revendication 1 ou 2, **caractérisé en ce que** chaque élément d'articulation (10) compte au moins deux trous de passage s'étendant dans le sens de son axe (11 a, 11 b) et **en ce que** le câble tendeur ou le fil tendeur (8) court sur un trajet allant au moins de l'extrémité proximale à l'extrémité distale de la chaîne articulée, et retour.

4. Dispositif à chaîne articulée selon l'une quelconque des revendications précédentes, **caractérisé en ce que** les éléments d'articulation (10 ; 110) sont fabriqués en plastique.

5. Dispositif à chaîne articulée selon la revendication 4, **caractérisé en ce que** des conducteurs électriques sont incorporés dans le plastique.

6. Dispositif à chaîne articulée selon l'une quelconque des revendications précédentes, **caractérisé en ce que** les éléments d'articulation (10 ; 110) présentent une structure superficielle renforçant la friction au niveau de leurs surfaces réciproquement opposées (13, 14 ; 113, 114).

7. Système de retenue, en particulier pour des fonctions médicales, comprenant un dispositif à chaîne articulée selon l'une quelconque des revendications précédentes, un élément de retenue (9 ; 109) conçu pour maintenir un objet, en particulier un outil ou un instrument médical, et un appareil pour chauffer et/ou refroidir l'élément à mémoire de forme (8 ; 115).

8. Système de retenue selon la revendication 9, **caractérisé en ce que** l'appareil pour chauffer et/ou refroidir comprend un chauffage électrique.
